# EUROPÄISCHE PATENTANMELDUNG

(11) **EP 4 606 357 A1**
(43) Veröffentlichungstag der Anmeldung: **27.08.2025**
(21) Anmeldenummer: 24159752.5
(22) Anmeldetag: 26.02.2024
(51) Int. Cl.: A61F 7/00, A61H 33/00

(54) **KÜHLANORDNUNG UND KÜHLVERFAHREN**

(71) Anmelder: Hampl, Trebst GbR, 50937 Köln (DE)
(72) Erfinder: TREBST, Tom-Nicolas, 20733 Köln (DE); HAMPL, Aurel, 50931 Köln (DE)
(74) Vertreter: Wunderlich & Heim Patentanwälte PartG mbB

(57) **Zusammenfassung**

Die Erfindung betrifft eine Kühlanordnung mit einem Behältnis, welches zum Aufnehmen eines Körpers in einer Flüssigkeit, insbesondere Wasser, ausgebildet ist, insbesondere eine Wanne oder Tonne, einer Kühleinrichtung, welche zum Kühlen der Flüssigkeit ausgebildet ist und mindestens einen Einlass für die Flüssigkeit und mindestens einen Auslass für die Flüssigkeit aufweist, wobei die Flüssigkeit bei Durchlauf durch die Kühleinrichtung von dem mindestens einen Einlass zu dem mindestens einen Auslass kühlbar ist und von dem Auslass zu dem Behältnis zuführbar ist, und einer Steuereinheit zum Steuern der Kühleinrichtung zum Einstellen zumindest der Temperatur der Flüssigkeit, welche dem Behältnis zugeführt wird.

## Beschreibung

Die Erfindung betrifft eine Kühlanordnung mit einem Behältnis, welches zum Aufnehmen eines Körpers in einer Flüssigkeit, insbesondere Wasser, ausgebildet ist, insbesondere eine Wanne oder Tonne, einer Kühleinrichtung, welche zum Kühlen der Flüssigkeit ausgebildet ist und mindestens einen Einlass für die Flüssigkeit und mindestens einen Auslass für die Flüssigkeit aufweist, wobei die Flüssigkeit bei Durchlauf durch die Kühleinrichtung von dem mindestens einen Einlass zu dem mindestens einen Auslass kühlbar ist und von dem Auslass zu dem Behältnis zuführbar ist, und einer Steuereinheit zum Steuern der Kühleinrichtung zum Einstellen zumindest der Temperatur der Flüssigkeit, welche dem Behältnis zugeführt wird, gemäß dem Anspruch 1.

Weiterhin betrifft die Erfindung ein Kühlverfahren, insbesondere mit erfindungsgemäßen einer Kühlanordnung, wobei ein Behältnis zum Aufnehmen eines Körpers, insbesondere eine Wanne oder Tonne, mit einer Flüssigkeit, insbesondere Wasser, gefüllt wird, die Flüssigkeit mit einer Kühleinrichtung mit mindestens einem Einlass für die Flüssigkeit und mindestens einem Auslass für die Flüssigkeit gekühlt wird, die Flüssigkeit bei Durchlauf durch die Kühleinrichtung von dem mindestens einen Einlass zu dem mindestens einen Auslass gekühlt wird und von dem Auslass dem Behältnis zugeführt wird, und die Kühleinrichtung von einer Steuereinheit zum Einstellen zumindest der Temperatur der Flüssigkeit, welche dem Behältnis zugeführt wird, gesteuert wird, gemäß dem Anspruch 15.

Für Kühlverfahren, insbesondere im Bereich von Wellness-Anwendungen, sind zuverlässige und sichere Kühltechnologien erforderlich. Insbesondere an warmen Tagen oder nach einer anstrengenden oder sportlichen Betätigung wird es als wohltuend empfunden, den Körper in einer kalten Flüssigkeit abzukühlen. Hierfür schon seit Langem spezielle Kühlvorrichtungen bekannt, welche ein sogenanntes Eisbad ermöglichen. Die Kühlung erfolgt dabei meistens durch die Zugabe von Eiswürfeln in eine Tonne oder eine Wanne, die mit Wasser gefüllt sind. Entscheidend dabei ist, dass die Kühlvorrichtung für ein dauerhaftes und steuerbares Absenken der Temperatur eines Körpers eines Nutzers geeignet ist, wobei Sicherheit vor allem Vorrang hat. Es ist bekannt, dass für die Sicherheit solcher Anwendungen, insbesondere bei Eisbädern, das Erfassen und langfristige Auswerten von Temperaturdaten sinnvoll ist.

Bisherige Kühlvorrichtungen zeichnen sich vor allem durch ihren großen Platzbedarf und eine komplizierte Bedienung aus, wobei häufig entsprechend geschultes Fachpersonal zur Anwendung vonnöten ist. Insbesondere das Bereitstellen von Eiswürfeln in großen Mengen ist mit erheblichem Aufwand verbunden.

Der Erfindung liegt die **Aufgabe** zugrunde, eine Kühlanordnung und ein Kühlverfahren bereitzustellen, welche eine schnelle, unkomplizierte und flexible Anwendung, insbesondere im häuslichen Bereich, ermöglichen.

Die Aufgabe wird nach der Erfindung durch eine Kühlanordnung mit den Merkmalen des Anspruchs 1 sowie durch ein Kühlverfahren mit den Merkmalen des Anspruchs 15 gelöst. Bevorzugte Ausführungsformen der Erfindung sind in den jeweils abhängigen Ansprüchen angegeben.

Eine Grundidee der Erfindung liegt in der Bereitstellung von Eisbädern im vorzugsweise häuslichen Bereich durch Nutzung eines geeigneten Behältnisses, etwa einer Badewanne oder eine Badetonne, und einer kompakten, mobilen Kühleinrichtung, wobei die Flüssigkeit beim Durchlauf durch die Kühleinrichtung gekühlt und in das Behältnis geleitet wird. Die Flüssigkeit kann auch jedes geeignete, strömungsfähige Kühlmedium sein, wobei im Sinne der Erfindung insbesondere eine Suspension oder theoretisch sogar ein Gas umfasst werden. Für die Kühleinrichtung ist eine Steuereinheit zum Steuern der Kühleinrichtung vorgesehen, womit die Temperatur der Flüssigkeit wunschgemäß verstellbar ist. Die Steuereinheit kann vorzugsweise als Regler ausgebildet sein. Die Kühleinrichtung kann in gewissem Umfang auch für ein Erhitzen der Flüssigkeit ausgebildet sein, insbesondere wenn die Temperatur einen bestimmten Grenzwert unterschreitet. So ist gewährleistet, dass die Kühlanordnung für ein schnelles und benutzerfreundliches Kühlen der Flüssigkeit auf eine eingestellte Temperatur geeignet ist.

Ein weiterer Grundgedanke der Erfindung liegt darin, ein Kühlverfahren bereitzustellen, wobei ein Behältnis mit einer Flüssigkeit, insbesondere Wasser, gefüllt wird und ein Kühlen dadurch erzielt wird, dass die Flüssigkeit zu einer Kühleinrichtung zum Kühlen geleitet wird. Anschließend wird die Flüssigkeit der Wanne zugeführt. Teil des Verfahrens ist es, dass die Kühleinrichtung zum Einstellen der Flüssigkeitstemperatur gesteuert wird.

Eine zweckmäßige Weiterbildung der Erfindung besteht darin, dass die Steuereinheit mit mindestens einer rechnergestützten, vorzugsweise mobilen Eingabeeinrichtung zum Eingeben von Daten und/oder Steuerbefehlen verbindbar ist. Die Verbindung kann hierbei drahtlos, beispielweise mit einem W-LAN-Netzwerk oder über ein Mobilfunknetzwerk erfolgen. Mit anderen Worten kann eine Verbindung zwischen der Steuereinheit und der mindestens einen rechnergestützten Eingabeeinrichtung bestehen, wobei die Eingabe von Daten auch einen Datenaustausch umfassen kann. Für die Verbindung mit einem drahtlosen Funknetzwerk kann die Steuereinheit insbesondere ein Smart Hub aufweisen. Besonders vorteilhaft ist es auch, wenn die Eingabeeinrichtung eine Benutzeroberfläche im oberen Bereich der Kühleinrichtung aufweist.

Besonders bevorzugt ist es, dass die rechnergestützte Eingabeeinrichtung ein Mobiltelefon und/oder einen Tabletcomputer, insbesondere mit einer grafischen Bedienoberfläche, umfasst. Die rechnergestützte Eingabeeinrichtung kann vorzugsweise ein Steuerprogramm, insbesondere eine App, aufweisen, mit der beispielsweise der Zeitpunkt einer bestimmten Zieltemperatur der Flüssigkeit von einem Nutzer eingestellt werden kann. Der Nutzer kann dabei einen Fragebogen zum individuellen Einstellen, insbesondere des Betriebsmodus, der Kühlanordnung durchlaufen. Die rechnergestützte Eingabeeinrichtung kann in einem bidirektionalen Datenaustausch mit der Steuereinheit stehen und beispielsweise Benachrichtigungen bei Erreichen bestimmter Zielwerte empfangen. Folglich kann die rechnergestützte Eingabeeinrichtung auch zum Empfangen und Anzeigen von Daten, insbesondere Echtzeitdaten, ausgebildet sein.

In einer bevorzugten Ausführungsform der Erfindung kennzeichnet sich die Kühlanordnung dadurch, dass mindestens ein Sensor zum Erfassen von Zuständen, insbesondere von Zustandsdaten des Körpers, vorgesehen ist, wobei der mindestens eine Sensor mit der Steuereinheit und/oder der rechnergestützten Eingabeeinrichtung verbindbar ist. Der Sensor kann als Wearable, insbesondere als Smartwatch, ausgebildet sein und mit der Steuereinheit und/oder der rechnergestützten Eingabeeinrichtung in drahtloser Verbindung zum Austausch von Daten stehen. Vorgesehen ist, dass der Sensor insbesondere zum Erfassen von Zustandsdaten des menschlichen Körpers, beispielsweise bei Durchführung einer Kältetherapie, ausgebildet ist. Unter Zustandsdaten im Sinne der Erfindung sind vorzugsweise Vitaldaten betreffend insbesondere die Schlafdauer und Schlafqualität, der Puls, der Blutdruck, die Ruheherzfrequenz, die Frequenz der Atmung und/oder die Herzvariabiliät zu verstehen. So können der optimale Zeitpunkt, die Dauer, die Frequenz und die Kälteintensität für die Kältetherapie personalisiert und durch eine langfristige Anwendung gesundheitliche Vorteile erzielt werden.

Insbesondere durch Ausbildung des Sensors als Wearable kann dem Nutzer ein Echtzeitfeedback, beispielsweise durch Überwachung des Pulses während des Eisbadens gegeben werden. Das Wearable kann mit der rechnergestützten Eingabeeinrichtung, vorzugsweise einem Smartphone, in kommunikationstechnischer Verbindung stehen und durch Benachrichtigungen den Nutzer darauf hinweisen, wenn bestimmte physiologische Ziele, insbesondere eine Körpertemperatur, erreicht wurden. Der Sensor kann etwa als Temperatursensor zur Messung der Hauttemperatur des Nutzers während des Eisbadens ausgebildet sein. Es kann so ein größtmöglicher Nutzen für den Anwender erreicht und eine übermäßige Kühlung mit möglichen Schäden vermieden werden.

Ebenso kann der Sensor zur kontinuierlichen Überwachung der Herzfrequenz während des Eisbadens ausgebildet sein. Die so gewonnenen Daten können zur weiteren Verarbeitung vorzugsweise an die Steuereinheit und/oder die Eingabeeinrichtung übermittelt werden. Mit dem Sensor wird somit die physiologische Belastung des Nutzers überwacht und einer möglichen Unterkühlung vorgebeugt. Ferner können Nutzer ihre Reaktion auf die Kältetherapie besser verstehen und die Erholung optimieren. Alternativ oder ergänzend dazu kann der Sensor als Pulsoximeter zum Erfassen der Sauerstoffsättigung im Blut ausgebildet sein.

Eine besonders zweckmäßige Weiterbildung ist es, dass der Sensor zum Ausgeben /und oder Versenden eines Alarms, insbesondere bei anhaltendem Puls unter 35 Schlägen pro Minute, ausgebildet ist. Dabei kann die Möglichkeit zum Aufheben des Alarms durch den Benutzer im Falle eines Fehlalarms vorgesehen sein. Der Sensor kann auch ausgebildet sein, bradykarde Zustände, insbesondere eine Herzfrequenz unter 35 Schläge pro Minute, als Vorwarnung zu erkennen. Hierbei ist es besonders vorteilhaft, wenn der Sensor zum Absenden eines automatischen Notrufs ausgebildet ist, falls der Alarm nicht storniert wird.

Eine weitere vorteilhafte Ausgestaltung der Erfindung besteht darin, dass die Kühleinrichtung eine Wärmabführeinheit, insbesondere einen Wärmetauscher und/oder eine Filtereinrichtung zum Filtern der Flüssigkeit umfasst. Die Wärmeabführeinheit kann einen Kompressor, einen Wärmetauscher und insbesondere ein Kühlmittel, beispielsweise R-134A, das mit Entropie arbeitet, aufweisen. Das Kühlmittel kann für einen Wechsel zwischen gasförmigen und flüssigen Phasen durch Kompression und Expansion ausgelegt sein. Die Wärmeabführeinheit kann als Kühlkörper, Lüfter und /oder Peltiermodul ausgebildet sein. Dadurch wird eine schnelle und effiziente Kühlung der Flüssigkeit sichergestellt.

Die Kühleinrichtung kann vorzugsweise einen Durchmesser von etwa 30 cm und eine Höhe von etwa 60 cm aufweisen. Vorteilhaft ist es, wenn die Grundfläche der Kühleinrichtung 0,25 m² nicht übersteigt.

Die Kühleinrichtung kann zusätzlich eine Pumpe aufweisen. Die Pumpe kann auswechselbar ausgebildet sein. Vorteilhaft ist es insbesondere, wenn die Pumpe mit einer Sensoreinheit verbunden ist, die zum Erfassen der Fließgeschwindigkeit der Flüssigkeit ausgestaltet ist. Ferner kann die Sensoreinheit zum Steuern der Pumpe ausgebildet sein. Hierdurch wird eine effiziente Fließgeschwindigkeit für das Wasser geschaffen, was sich positiv auf die Kühlwirkung auswirkt. Vorteilhaft ist es auch, wenn die Wasserpumpe schwingungsdämpfende Elemente aufweist. Alternativ oder ergänzend dazu kann die Kühleinrichtung einen Wassertemperatursensor, einen leisen Lüfter mit integriertem Feinfilter und/oder ein ausziehbares wasserdichtes Kabel zur Energieversorgung aufweisen. Das Kabel kann vorzugsweise ausziehbar mit bis zu etwa 4 Metern Ausziehlänge ausgebildet sein.

Eine besonders zweckmäßige Weiterbildung der Erfindung besteht darin, dass ein Datenspeicher für die Steuereinheit vorgesehen ist, wobei in dem Datenspeicher für ein oder mehrere Nutzungen der Kühlanordnung Daten speicherbar sind, insbesondere zur Temperatur, Nutzungs- / Kühldauer, Nutzungszeitpunkt und/oder Daten mindestens eines Nutzers der Kühlanordnung. Der Datenspeicher kann insbesondere auch zur sicheren Speicherung von Grunddaten zur Person und/oder Gesundheitsdaten ausgebildet sein. Es können insbesondere vorab Grunddaten des Nutzers, etwa zu Geschlecht, Alter, Gewicht, Körperfettanteil, Fitnesszustand etc eigegeben sein, welche so von der Steuereinheit für eine individuelle Anwendung berücksichtigt werden können.

Besonders zweckdienlich ist es hierbei, wenn der Datenspeicher mit der rechnergestützten Eingabeeinrichtung und/oder dem Sensor zum Austausch von Daten in kommunikationstechnischer Verbindung steht. So können beispielsweise Nutzerdaten, insbesondere anthropometrische Daten, von einer App zum Weiterverwenden übernommen werden. Insbesondere können Starttemperatur und Kühldauer an die individuellen Bedürfnisse und den Trainingsstand des Nutzers angepasst werden. Vorzugsweise ist der Datenspeicher derart ausgebildet, dass Nutzerdaten, insbesondere personalisierte Nutzerdaten betreffend beispielsweise Nutzungsdauer, Kühldauer, Nutzungszeitpunkt und/oder Temperaturverlauf exportierbar sind, um so beispielsweise Trainingspläne mit geschultem Fachpersonal zu erstellen.

Nach einer Ausgestaltung der Erfindung ist der Datenspeicher als auswechselbare, mobile Speichereinheit ausgebildet, die der Kühlanordnung entnommen werden kann. Gemäß einer anderen Ausführungsvariante ist der Datenspeicher fest in der Kühlanordnung verbaut und kann durch Herstellen einer Datenverbindung ausgelesen werden. Zweckmäßig kann es ebenfalls ein, wenn der Datenspeicher zum Speichern von Daten aus dem Sensor und/oder der mindestens einen rechnergestützten Eingabeeinrichtung, insbesondere von Fitnessdaten, Körperdaten und Echtzeitvitalparametern ausgebildet ist.

Besonders bevorzugt ist es weiterhin, dass die Steuereinheit ausgebildet ist, basierend auf in dem Datenspeicher gespeicherten Daten eine Empfehlung für Eingaben zu einer weiteren oder zukünftigen Nutzung der Kühlanordnung, insbesondere betreffend Temperatur, Nutzung/Kühldauer, Nutzungszeitpunkt, insbesondere betreffend einen mindestens einen Nutzer, auszugeben, insbesondere über eine Anzeige oder eine vorzugsweise mobile Ausgabeeinrichtung. Gemäß der Erfindung kann die mobile Ausgabeeinrichtung dabei der mindestens einen rechnergestützten Eingabeeinrichtung entsprechen. Die Empfehlung kann insbesondere mit einem Programm für maschinelles Lernen, vorzugsweise einer Kl, errechnet werden. Dabei kann vorgesehen sein, insbesondere gespeicherte Daten aus der Eingabeeinrichtung und/oder aus dem Sensor heranzuziehen. Vorteilhaft ist es, wenn für die Empfehlung Grunddaten zur Person, beispielsweise Alter, Körpergröße und Gewicht, Geschlecht sowie zum Fitness- und Gesundheitszustand in die Berechnung einfließen.

Gemäß einer Weiterbildung der Erfindung ist vorgesehen, dass zwischen mindestens einem Sensor und der mindestens einen rechnergestützten Eingabeeinrichtung eine Datenverbindung herstellbar ist, mit der Sensordaten an die rechnergestützte Eingabeeinrichtung übertragbar sind. Beispielsweise können so Fitnessdaten von einer als Smartphone ausgebildeten Eingabeeinrichtung mit Wearable-Daten in einer entsprechenden App kombiniert werden und so einen umfassenden Überflick über den Regenerationsfortschritt bieten. Die Steuereinheit kann dabei ebenfalls mit der App verbindbar sein.

Alternativ oder ergänzend dazu kann es nach einer Weiterentwicklung der Erfindung vorteilhaft sein, dass die Steuereinheit, die Eingabeeinrichtung und/oder die Ausgabeeinrichtung ein Datenverarbeitungsprogramm mit maschinellem Lernen aufweist. Das Datenverarbeitungsprogramm kann dabei als künstliche Intelligenz ausgebildet sein und wird fortan als KI bezeichnet. Nachfolgend werden Ausführungsvarianten der KI näher beschrieben.

Die KI kann ausgebildet sein, Daten von der Steuereinheit, der Eingabeeinrichtung und/oder des Sensors in Echtzeit auszuwerten, um in lernender Weise progressive und motivierende Empfehlungen betreffend Temperatur, Nutzungs-/Kühldauer, Nutzungszeitpunkt, insbesondere betreffend mindestens einen Nutzer, zu erstellen und über eine Anzeige auszugeben. So können etwa langfristig herausragende Trainingsergebnisse des Nutzers erzielt werden. Zusätzlich kann vorgesehen sein, dass die KI die genannten Daten über einen längeren Zeitraum sammelt, um langfristige vorzugsweise personenbezogene Trends zu identifizieren. Grundsätzlich kann die KI als App für mobile Endgeräte, insbesondere für die rechnergestützte Eingabeeinrichtung und/oder den Sensor ausgebildet sein, zweckmäßig ist es aber auch, wenn die KI als Computerprogramm einer externen Rechnereinheit ausgebildet ist. Vorteilhaft ist es ebenso, wenn die KI basierend auf Nutzerantworten auf einem Fragebogen, historischen Nutzungsdaten und/oder Grunddaten zur Person individuelle Empfehlungen erstellen kann.

Eine weitere vorteilhafte Ausgestaltung der Erfindung besteht darin, dass die KI ausgebildet ist, Daten aus Forschungsergebnissen zur Kältebehandlung heranzuziehen und daraus Empfehlungen an den Nutzer abzuleiten. Weiterhin kann die KI ausgebildet sein, basierend auf Zieleingaben und Trainingsfortschrittsdaten personalisierte Empfehlungspläne zu erstellen. Vorzugsweise können dabei Benutzerfeedback-Fragebögen verwendet werden. Zweckmäßig ist es auch, wenn die KI zur adaptiven Steuerung der Kühleinrichtung, insbesondere der Wärmeabführeinheit, ausgebildet ist. Alternativ oder ergänzend dazu kann die KI ausgestaltet sein, menschliche Stressreaktionen wir erhöhten Puls oder erhöhte Sauerstoffsättigung während des Eisbades zu analysieren und darauf aufbauend dem Nutzer Atemübungen zu empfehlen. Vorzugsweise erfolgt dabei die Anzeige visuell beispielsweise auf dem Smartphone oder dem Wearable.

Weiterhin kann die KI ausgebildet sein, die Kühleinrichtung individuell an den Nutzer, basierend auf Nutzerdaten, insbesondere Trainingsdaten, physiologischen und Vitaldaten und Nutzereinstellungen anzupassen. Darüber hinaus kann die KI Prognosen über die individuell benötigten Erholungszeit nach verschiedenen Trainingseinheiten erstellen. In diesem Zusammenhang kann es vorteilhaft sein, wenn die KI zur Maximierung der Erholung ausgebildet ist, und den Nutzer zum besten Zeitpunkt für das nächste Eisbad zu benachrichtigen. Es können so Wohlbefinden, Fitnesszustand und/oder eine Widerstandsfähigkeit gegen Krankheiten, etwa Erkältungen, gesteigert werden.

Aus Sicherheitsgründen hat es sich als besonders vorteilhaft erwiesen, wenn die KI ausgebildet ist, ein Übertraining anhand von individuellen Trainingsdaten frühzeitig zu erkennen. Im Falle eines Übertrainings kann die KI den Sportler, insbesondere basierend auf wissenschaftlichen Erkenntnissen, warnen. Gemäß einer Ausführungsvariante kann die KI in der Lage sein, Erholungspläne auf Basis von Vitaldaten zur Person zu erstellen. Vitaldaten zur Person können insbesondere Daten zur Schlafdauer und Qualität, Atemfrequenz und Ruhepuls sein. Vorzugsweise können beim Erstellen der Erholungspläne auch die Trainingshistorie und der individuelle Erholungsbedarf miteinfließen.

Zur Reduktion von HRV kann die KI zusätzlich ausgebildet sein, langfristige Trends durch Datenanalyse über mehrere Einzelnutzungen zu identifizieren. Insbesondere betrifft dies die Anpassung des Pulses auf die Kälteexposition. Hierbei ist es besonders zweckdienlich, wenn Daten aus verschiedenen Quellen, insbesondere dem Sensor und/oder der rechnergestützten Eingabeeinrichtung kombiniert werden. Das heißt, dass die KI vorzugsweise derart ausgestaltet ist, Daten aus verschiedenen Quellen, insbesondere betreffend das Nutzungsverhalten, persönliches Feedback, Bedürfnisse, Vitalparameter und Vitalparameterhistorie, Fitnessdaten, Health-Apps und/oder anthropometrische Daten aus Fragebögen zu verknüpfen und Trainingsplananpassungen automatisiert vorzunehmen und optimale Nutzungszeitpunkte auszugeben.

Eine besonders vorteilhafte Ausführung wird nach einer Weiterbildung der Erfindung dadurch erzielt, dass die Kühlanordnung ausgebildet ist, die Flüssigkeit mit Ozon anzureichern. Dazu kann beispielsweise ein Schlauch oder ein Rohr verwendet werden. Ebenso kann ein Filter in der Kühlanordnung, insbesondere vor dem Einlass der Kühleinrichtung, angeordnet sein. Damit können beispielsweise Grobpartikel und andere Verunreinigungen aus der Flüssigkeit gereinigt werden.

Nach einer Weiterbildung der Erfindung kann die Kühlanordnung einen zusätzlichen Einlass aufweisen, der ausgebildet ist, die Flüssigkeit mit Zusatzmitteln anzureichern. Hierdurch können beispielsweise Gase oder Badezusatzstoffen in die Flüssigkeit eingebracht werden.

Eine besonders zweckdienliche Ausführungsvariante der Erfindung besteht darin, dass die Kühlanordnung mindestens eine Zuführung aufweist, die ausgebildet ist, die Flüssigkeit zum Einlass der Kühleinrichtung zu leiten. Die Zuführung kann insbesondere als Saug- und Zuganschluss an einen Wasserauslass, insbesondere eine Badewannenarmatur, angeschlossen sein. Die Zuführung kann als Schlauch, insbesondre als Gummischlauch mit isolierenden und schnelltrocknenden Eigenschaften, ausgebildet sein. Vorteilhaft ist es hierbei, wenn der Schlauch auf bis zu etwa dem Dreifachen seiner Länge ausziehbar ist.

Nach einer Weiterbildung der Erfindung weist die Zuführung mindestens einen elastischen Schlauchabschnitt auf, der mit dem Wasserauslass, insbesondere einer Badewannenarmatur oder einem Wasserhahn, verbindbar ist. Der Anschluss kann vorzugsweise durch ein Überstülpen des Schlauchs an dem Wasserhahn erfolgen. Hierdurch wird in einfacher Weise eine besonders sichere Verbindung hergestellt.

Die als Schlauch ausgebildete Zuführung kann vorzugsweise mehrere, insbesondere drei, Lagen aufweisen. Hierbei kann ein 3750D-Polyesterstoff und/oder ein 3750D-Polyestergemisch mit Glaswolle oder Steinwolle für einen besonders guten Isolierungseffekt verwendet werden. Eine Lage kann vorzugsweise als Latexschicht ausgebildet sein. Beispielsweise können drei Schichten umfassend eine langlebige wasserfeste Innenschicht zum Transport der Flüssigkeit, eine Mittelschicht zur thermischen Isolierung des Wassers und zur Kondensationsvermeidung sowie einer schnelltrocknenden Außenschicht vorgesehen sein.

Eine weitere Ausführungsvariante der Erfindung besteht darin, dass die Zuführung mindestens drei Endstücke aufweist, wobei mindestens ein Endstück zum Ableiten der Flüssigkeit aus dem Behältnis ausgebildet ist. Als besonders vorteilhaft hat es sich herausgestellt, wenn ein Endstück mit dem Einlass der Kühleinrichtung verbunden ist, wobei die Verbindung insbesondere durch einen ¾-Zoll-Schraubverschluss erfolgen kann. Ein zweites Endstück ist mit dem Wasserauslass, vorzugsweise einer Badewannenarmatur, verbunden und ein drittes Endstück ist zum Ableiten der Flüssigkeit aus dem Behältnis vorgesehen. Bei dieser Ausgestaltung kann das zweite Endstück als Schlauch, vorzugsweise mit einer Latexinnenschicht, ausgebildet sein, der über den Wasserauslass ziehbar ist, und mit einem Schließsystem, beispielsweise einem Verschlussring befestigbar ist. Die Zuführung mit drei Endstücken kann als Y-Verzweigung ausgebildet sein, wobei die Flüssigkeit mittels einer Verstelleinrichtung, insbesondere einer Klappe, entweder von dem Wasserauslass zum Einlass der Kühleinrichtung oder aus dem Behältnis zu dem Einlass der Kühleinrichtung leitbar ist. Eine derart ausgebildete Zuführung soll eine Wasserzufuhr zu der Kühleinrichtung über zwei Wege ermöglichen, wahlweise von dem Wasserauslass oder von der Flüssigkeit, die sich in dem Behältnis befindet.

Gemäß einem weiteren Aspekt der Erfindung weist die Kühlanordnung mindestens eine Rückführung auf, die ausgebildet ist, Flüssigkeit aus der Kühleinrichtung abzuleiten. Die Rückführung kann ebenso wie die Zuführung ausgestaltet sein, insbesondere als mehrlagiger Schlauch mit schnelltrocknenden und isolierenden Eigenschaften. Vorteilhaft ist es, wenn die Rückführung auf bis zu etwa dem Dreifachen ihrer Länge ausziehbar ist. Die Rückführung dient der Rückführung der gekühlten Flüssigkeit aus der Kühleinrichtung in das Behältnis. Auf diese Weise kann ein Kühlkreislauf hergestellt werden, wobei die Flüssigkeit aus dem Behältnis über die Zuleitung in die Kühleinrichtung gepumpt wird, um die dort gekühlte Flüssigkeit über die Rückführung von der Kühleinrichtung zurück in das Behältnis gelangt. Dabei kann ein offenes Endstück der Rückführung mit einem Netz und einen O-Ring vorgesehen sein.

Als besonders vorteilhaft hat es sich nach einer Weiterbildung der Erfindung herausgestellt, dass die Rückführung und/oder die Zuführung mindestens ein Fixierelement, insbesondere einen Saugnapf, aufweisen. Damit kann beispielsweise die Rückführung während des Ableitens der Flüssigkeit aus der Kühleinrichtung an einer Innenwand des Behältnisses gehalten werden. Die Installation kann durch einfaches Drücken des Saugnapfes auf die Oberfläche erfolgen. Hierdurch wird ein unkontrolliertes Verschwenken der Rückführung und/oder der Zuführung während des Betriebs der Kühlanordnung vermieden.

Nach einer Weiterbildung der Erfindung ist mindestens ein Koppelelement vorgesehen, wobei das Koppelelement zwei Anschlussstücke aufweist, die zum verlustfreien Überleiten der Flüssigkeit, insbesondere aus einer Wasserleitungsarmatur, ausgebildet sind. Die Anschlussstücke können als zylindrische Endstücke mit äußeren Riffelungen ausgebildet sein. Vorzugsweise können die Anschlusstücke durch eine Drehdrückbewegung mit den elastischen Schlauchendstücken verbunden werden. Ein Lösen kann durch Drehen in entgegengesetzte Richtung erfolgen. Das Koppelelement kann insbesondere zur Verbindung der Kühlanordnung mit einer Wasserleitungsarmatur und/oder zum Anschluss der Zu- beziehungsweise Rückführungen an die Kühleinrichtung verwendet werden. Ebenso kann das Koppelelement zum Verbinden zweier elastischer Schlauchendstücke vorgesehen sein.

Gemäß einer Weiterbildung der Erfindung weist die Kühlanordnung eine thermische Isolierschicht, insbesondere eine Abdeckplane, zum Abdecken einer offenen Fläche des Behältnisses auf. Vorzugsweise kann die thermische Isolierschicht fest mit dem Behältnis verbunden werden. Die thermische Isolierschicht kann darüber hinaus Öffnungen, insbesondere für einen oberen Körperbereich, aufweisen. Mit der Isolierschicht soll ein Wärmeaustausch zwischen einer Flüssigkeit im Behältnis und der Umgebung reduziert werden.

Nach einer Ausführungsvariante der Erfindung weist die Isolierschicht ein Sicherungselement zum Fixieren auf, wobei für ein Abnehmen der Isolierschicht das Sicherungselement in spezieller Weise betätigt werden muss. Das Sicherungselement kann vorzugsweise als Kindersicherung ausgebildet sein. Ebenso soll ein unbeabsichtigtes Verrutschen der Isolierschicht und damit ein Angleichen der Flüssigkeitstemperatur mit der Umgebungstemperatur vermieden werden.

Die erfindungsgemäße Kühlanordnung, insbesondere wie zuvor beschrieben, kann zur Durchführung des erfindungsgemäßen Kühlverfahrens eingesetzt werden. Dabei können die zuvor beschriebenen Vorteile erzielt werden. Die Kühlanordnung kann für einen Körper, insbesondere eines Menschen oder Tieres, eingesetzt werden, um insbesondere das Wohlbefinden zu steigern.

Die Erfindung wird nachfolgend anhand von Ausführungsbeispielen, die schematisch in den Zeichnungen dargestellt sind, näher erläutert. In den Zeichnungen zeigen:
- Fig. 1: eine schematische Querschnittsdarstellung einer erfindungsgemäßen Kühleinrichtung mit Pumpe und Wärmeabführeinheit;
- Fig. 2: eine schematische Draufsicht einer erfindungsgemäßen Kühlanordnung;
- Fig. 3: eine schematische Seitendarstellung einer erfindungsgemäßen Kühlanordnung;
- Fig. 4: eine schematische Seitendarstellung einer erfindungsgemäßen Kühlanordnung in horizontaler Ausführung;
- Fig. 5: eine schematische Seitendarstellung einer erfindungsgemäßen Kühlanordnung in vertikaler Ausführung;
- Fig. 6: einen schematische Querschnittsdarstellung eines erfindungsgemäßen Behältnisses mit einer erfindungsgemäßen Zuführung;
- Fig. 7: eine schematische Seitendarstellung einer erfindungsgemäßen Kühlanordnung mit Rückführung; und
- Fig. 8: eine schematische Querschnittsdarstellung eines erfindungsgemäßen Koppelelements.

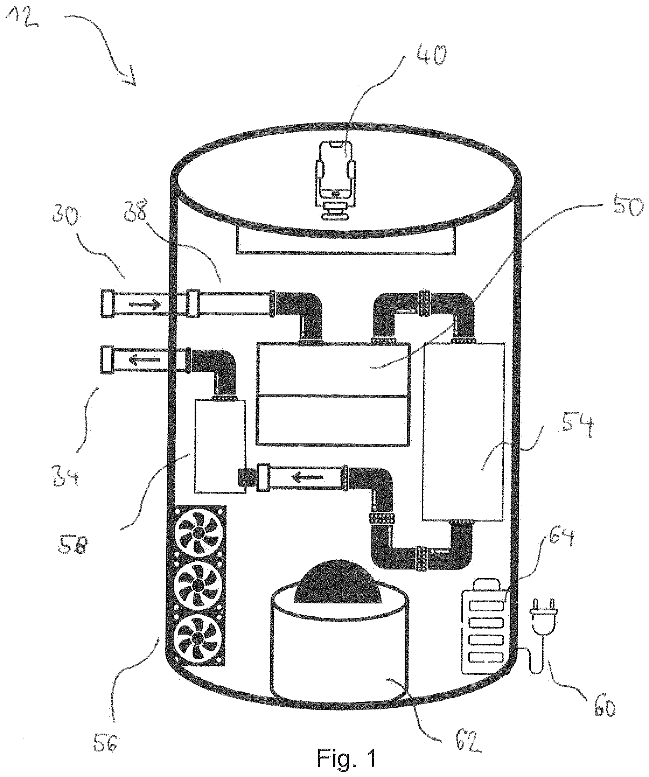

Fig. 1 zeigt eine schematische Querschnittsdarstellung einer erfindungsgemäßen Kühleinrichtung 12 mit zylindrischer Grundform. Grundsätzlich kann die Kühleinrichtung 12 jede Form aufweisen, beispielsweise auch eine rechteckige Grundform, die sich etwa unter den Gesichtspunkten der Handhabung, Wärmeabgabe und Lagerung als vorteilhaft erweist. Vorteilhaft ist es, wenn die Kühleinrichtung Spritzwasser geschützt ist oder wasserdichte Eigenschaften aufweist. Damit eignet sich die erfindungsgemäße Kühleinrichtung 12 besonders für einen Einsatz in nassfeuchten Umgebungen, insbesondere in einem Badezimmer. Die gezeigte Kühleinrichtung kann eine Wärmeabführeinheit 50, insbesondere einen Wärmetauscher und eine Pumpe 54 aufweisen, wobei die Pumpe 54 die zu kühlende Flüssigkeit durch die Kühleinrichtung entlang eines Kühlweges pumpt. Die Pumpe 54 kann zur Steuerung mindestens einen Sensor, insbesondere einen Wassersensor, aufweisen. Dabei kann die Pumpe 54 so ausgestaltet sein, den Pumpvorgang erst bei Wasserkontakt zu starten. Ein Schutzschalter kann eine Abschaltung bewirken, wenn Feuchtigkeit an einer falschen Stelle der Kühleinrichtung 12 detektiert wird.

Der Kühlweg kann wie in Figur 1 gezeigt so ausgestaltet sein, dass die Flüssigkeit am Einlass 30 zunächst mit einer Sensoreinheit 38 in Kontakt kommt, wobei die Sensoreinheit 38 zum Erfassen der Fließgeschwindigkeit und/oder der Flüssigkeitstemperatur ausgebildet sein kann. Gemäß der in Fig. 1 gezeigten Ausführungsvariante gelangt die Flüssigkeit, insbesondere Wasser, im weiteren Verlauf zu der Wärmeabführeinheit 50. Die Wärmeabführeinheit 50 kann ausgebildet sein, der Flüssigkeit Wärme zu entziehen und an die Umgebung abzugeben. Alternativ oder ergänzend dazu kann die Wärmeabführeinheit 50 auch zum Zuführen von Wärme ausgelegt sein.

Im nächsten Schritt kann die Flüssigkeit zu einer Pumpe 54 und von dort weiter zu einer Filtereinrichtung 58 geleitet werden. Besonders zweckmäßig ist es dabei, wenn die Filtereinrichtung 58 zum Reinigen der Flüssigkeit von Fremdpartikeln oder Stoffen ausgelegt ist. In der in Fig. 1 gezeigten Ausführungsvariante ist es vorgesehen, dass die Flüssigkeit im Anschluss von der Filtereinrichtung 58 zu einem Auslass 34 der Kühleinrichtung 12 geleitet wird. Wie in Fig. 1 gezeigt, kann die Kühleinrichtung 12 zusätzlich mindestens einen elektrischen Lüfter 56 zum effizienten Ableiten von Wärme und einen Datenspeicher 62 aufweisen.

Es hat sich als besonders vorteilhaft herausgestellt, wenn an der Kühleinrichtung 12 eine Haltevorrichtung 40, wie in Fig. 1 dargestellt, angebracht ist. Die Haltevorrichtung dient zum Befestigen einer mobilen Eingabeeinrichtung 42, vorzugsweise eines Smartphones, an der Kühleinrichtung 12.

Für eine flexible und unkomplizierte Energieversorgung der erfindungsgemäßen Kühlanordnung 10 kann ein ausziehbares Kabel 60 mit einem Netzteil 64 vorgesehen sein. Die Kühleinrichtung 12 kann für einen sicheren Stand mit mindestens einem Standfuß und/oder einer Gummimatte, vorzugsweise an der Unterseite, ausgestaltet sein. Ebenso können für den Transport der erfindungsgemäßen Kühleinrichtung 12 ein oder mehrere Tragegriffe, vorzugsweise im oberen Bereich, vorgesehen sein.

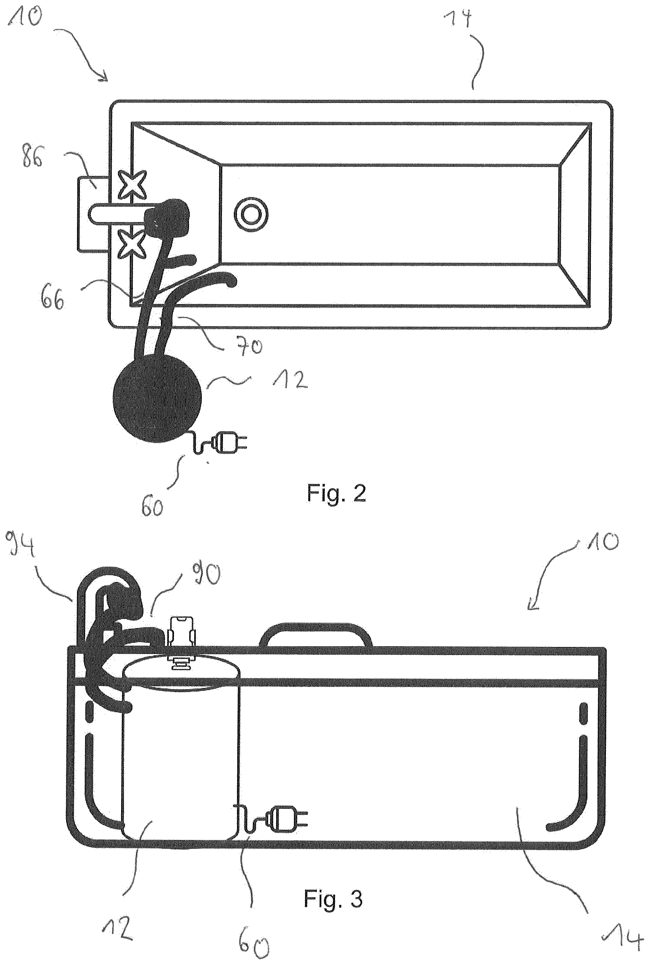

Fig. 2 zeigt eine schematische Draufsicht der erfindungsgemäßen Kühlanordnung 10 mit Kühleinrichtung 12 und Behältnis 14. Wie in Fig. 2 gezeigt, kann die Kühleinrichtung 12 im Seitenbereich des Behältnisses 14 angeordnet sein. Das Behältnis 14 kann wie in Fig. 2 gezeigt, einen Wasserauslass 34, vorzugsweise an der Unterseite, zum Ablassen der Flüssigkeit und eine Wasserleitungsarmatur 86, insbesondere eine Badewannenarmatur, aufweisen. Dabei kann vorgesehen sein, dass die Kühleinrichtung 12 mit einer Zuführung 66 mit der Wasserleitungsarmatur 86 zum Durchleiten der Flüssigkeit, vorzugsweise Wasser, verbunden ist. Die Zuführung 66 kann, wie in Fig. 2 gezeigt, zusätzlich ein offenes Endstück aufweisen, das vorzugsweise in das Behältnis 14 hineinragt und mit der sich dort befindlichen Flüssigkeit in Berührung kommt. Damit kann die Flüssigkeit sowohl aus der Wasserleitungsarmatur 86 als auch direkt aus dem Behältnis 14 in die Kühleinrichtung 12 geleitet werden. Die Kühlanordnung 10 kann zum Ableiten der Flüssigkeit 18 aus der Kühleinrichtung 2 eine Rückführung 70 aufweisen, die beispielsweise als elastischer Gummischlauch ausgebildet sein kann.

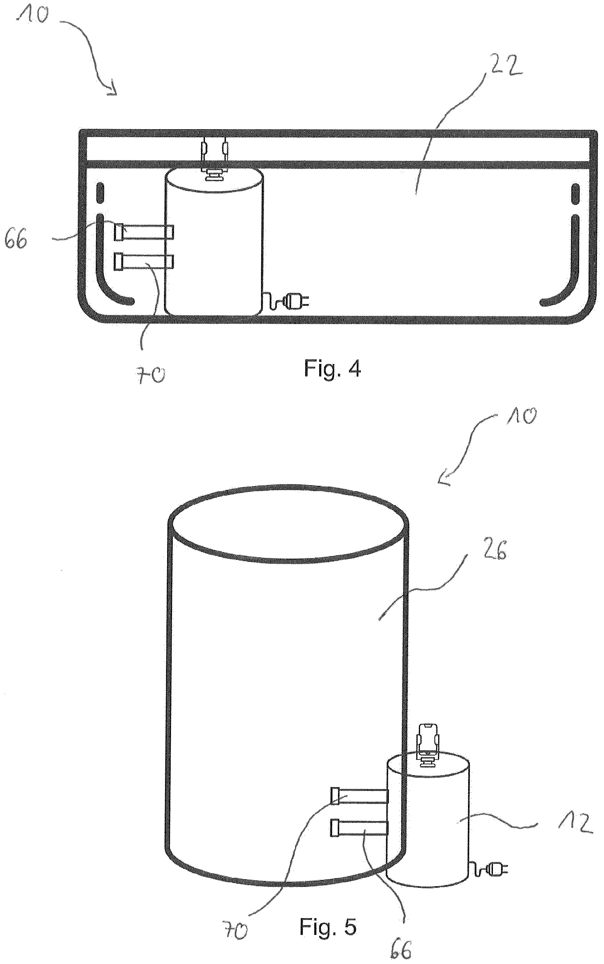

Fig. 3 und Fig. 4 zeigen jeweils eine schematische Seitendarstellung der erfindungsgemäßen Kühlanordnung 10. In Fig. 3 ist dargestellt, dass das Behältnis 14 mindestens einen Haltegriff für eine einfache Handhabung und einen einfachen Transport aufweisen kann. Grundsätzlich kann das Behältnis 14 von beliebiger Größe und Form sein. Für einen besonders flexiblen Einsatz kann das Behältnis 14 als aufblasbares Becken ausgeführt sein. Alternativ dazu kann das Behältnis 14 aber auch als Wanne 22, Tonne 26 oder Pool, insbesondere Whirlpool, insbesondere aus Holz, ausgebildet sein. Ebenso kann das Behältnis 14 als Zuber ausgebildet sein. Besonders vorteilhaft ist es, wenn die Kühleinrichtung 12, wie in Fig. 3 gezeigt, über flexibel windbare Schläuche 90 mit dem Behältnis 14 verbunden sind. Die Kühleinrichtung 12 kann aber auch über feste Rohrleitungen zum Leiten der Flüssigkeit 18 mit dem Behältnis 14 verbunden sein.

Die Fig. 5 zeigt eine schematische Seitenansicht der erfindungsgemäßen Kühlanordnung 10, wobei das Behältnis 14, insbesondere eine Tonne 26, vertikal ausgerichtet ist. Wie in Fig. 4 verbildlicht, kann das Behältnis 14 jedoch auch horizontal ausgerichtet sein. Die Kühleinrichtung 12 ist vorzugsweise in einem unmittelbaren Seitenbereich des Behältnisses 14 angeordnet. Alternativ kann sich die Kühleinrichtung 12 auch in räumlicher Distanz zu dem Behältnis 14 befinden und mit einer speziellen Rückführung, insbesondere einem Rohrsystem, mit dem Behältnis 14 verbunden sein. Vorteilhaft ist es hierbei, wenn der Einlass 30 der erfindungsgemäßen Kühleinrichtung 12 direkt mit einer Flüssigkeitszufuhr, vorzugsweise einem Wasseranschluss, verbunden ist. Die in den Figuren 4 und 5 dargestellte Kühlanordnung 10 kann auch so ausgestaltet sein, dass sich die Kühleinrichtung 12 innerhalb des Behältnisses 14 befindet und/oder fest mit dem Behältnis 14 verbaut ist.

Das Behältnis 14 kann ferner Einstiegs- und Ausstiegshilfen aufweisen. Ebenso können in dem Behältnis 14 Sitz- und/oder Liegeeinrichtungen für einen verbesserten Nutzerkomfort ausgebildet sein. Zusätzlich zu der Flüssigkeit 18 kann das Behältnis 14 mit Eis, insbesondere mit Eiswürfeln, befüllt sein.

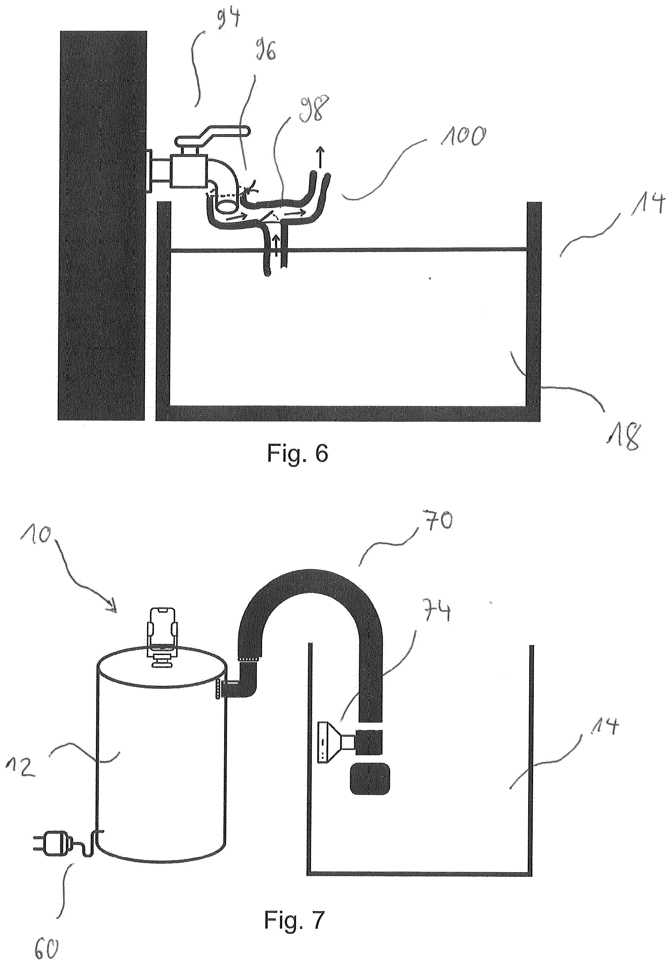

Die Fig. 6 zeigt eine schematische Querschnittsdarstellung der erfindungsgemäßen Zuführung 66. Wie in Fig. 6 dargestellt, kann die erfindungsgemäße Zuführung 66 als Y-Verzweigung 100 ausgebildet sein, wobei die Y-Verzweigung 100 als Schlauch 90 mit vorzugsweise drei Endstücken ausgebildet sein kann. Dabei kann ein Endstück in das Behältnis 14 hineinragen vorzugsweise mit der Flüssigkeit 18 in Verbindung stehen. Ein zweites Endstück kann wie in Fig. 6 gezeigt, mit einem Wasserauslass 94, insbesondere einer Armatur, durch Überstülpen des elastischen Schlauches 90 verbunden sein. Vorzugsweise kann ein Verschlussring 96 zur Befestigung der erfindungsgemäßen Zuführung an den Wasserauslass 34 vorgesehen sein. Durch das Einbringen einer Verstelleinrichtung 98, insbesondere einer Klappe, kann die Zuführung 66 wahlweise zum Durchleiten der Flüssigkeit 18 zwischen zwei Endstücken vorgesehen sein. Die Y-Verzweigung 100 kann insbesondere derart ausgestaltet sein, dass ein Durchlaufen der Flüssigkeit 18 von dem Wasserauslass 94 direkt in das Behältnis 14 möglich ist. Dadurch kann das Befüllen des Behältnisses 14 mit der Flüssigkeit 18 vor beziehungsweise während der Kühlung sichergestellt werden. Alternativ oder ergänzend dazu kann die erfindungsgemäße Kühlanordnung 10 auch ohne Pumpe 54 ausgebildet sein, wobei lediglich der Wasserauslassdruck des Wasserauslasses 94 genutzt wird.

Fig. 7 zeigt eine schematische Querschnittsdarstellung der erfindungsgemäßen Kühlanordnung 10, wobei die Kühleinrichtung 12 eine gekrümmte Rückführung 70 aufweist. Die Krümmung kann vorzugsweise U-förmig sein. Wie in Fig. 7 ausgeführt, kann die Rückführung als Rohr ausgestaltet sein. Besonders vorteilhaft ist es, wenn die Rückführung 70 ein Fixierelement 74, insbesondere einen Saugnapf, zum Fixieren der Rückführung 70 aufweist. Damit kann die Rückführung 70 auch während des Kühlbetriebes neu positioniert werden.

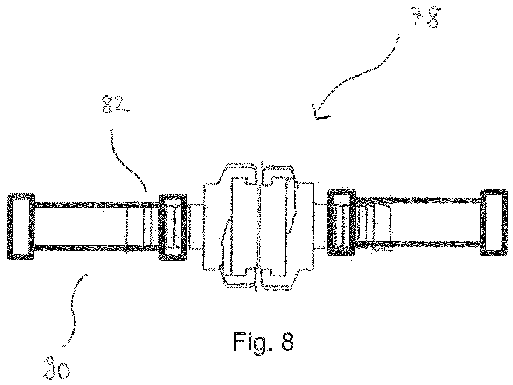

Fig. 8 zeigt eine schematische Seitendarstellung eines erfindungsgemäßen Koppelelements 78. Das Koppelelement 78 kann zwei Anschlussstücke 82 aufweisen, die mit dem Schlauch 90 verbindbar sind. Die Anschlussstücke 82 können grundsätzlich jede Form aufweisen, besonders bevorzugt ist es jedoch, wenn die Anschlussstücke 82 zylindrisch mit seitlichen Riffelungen ausgebildet sind. Eine besonders sichere Verbindung kann zwischen einem elastischen Schlauch 90 und dem Anschlussstück 82 durch ein Überziehen beziehungsweise überstülpen des Schlauches 90 hergestellt werden. Vorteilhaft ist es dabei, wenn die Anschlussstücke 82 mindestens einen sich zum Ende hin verjüngenden Bereich aufweisen.

## Patentansprüche

1. Kühlanordnung (10) mit
- einem Behältnis (14), welches zum Aufnehmen eines Körpers in einer Flüssigkeit (18), insbesondere Wasser, ausgebildet ist, insbesondere eine Wanne (22) oder Tonne (26),
- einer Kühleinrichtung (12), welche zum Kühlen der Flüssigkeit (18) ausgebildet ist und mindestens einen Einlass (30) für die Flüssigkeit (18) und mindestens einen Auslass (34) für die Flüssigkeit (18) aufweist, wobei die Flüssigkeit (18) bei Durchlauf durch die Kühleinrichtung (12) von dem mindestens einen Einlass (30) zu dem mindestens einen Auslass (34) kühlbar ist und von dem Auslass (34) zu dem Behältnis (14) zuführbar ist, und
- einer Steuereinheit zum Steuern der Kühleinrichtung (12) zum Einstellen zumindest der Temperatur der Flüssigkeit (18), welche dem Behältnis (14) zugeführt wird.

2. Kühlanordnung (10) nach Anspruch 1,
**dadurch gekennzeichnet,**
- **dass** die Steuereinheit mit mindestens einer rechnergestützten, vorzugsweisen mobilen Eingabeeinrichtung (42) zum Eingeben von Daten und/oder Steuerbefehlen verbindbar ist.

3. Kühlanordnung (10) nach Anspruch 2,
**dadurch gekennzeichnet,**
- **dass** die rechnergestützte Eingabeeinrichtung (42) ein Mobiltelefon und/oder einen Tabletcomputer, insbesondere mit einer graphischen Bedienoberfläche, umfasst.

4. Kühlanordnung (10) nach einem der Ansprüche 1 bis 3,
**dadurch gekennzeichnet,**
- **dass** mindestens ein Sensor zum Erfassen von Zuständen, insbesondere von Zustandsdaten des Körpers, vorgesehen ist, wobei der mindestens eine Sensor mit der Steuereinheit und/oder der rechnergestützten Eingabeeinrichtung (42) verbindbar ist.

5. Kühlanordnung (10) nach einem der Ansprüche 1 bis 4,
**dadurch gekennzeichnet,**
- **dass** die Kühleinrichtung (12) eine Wärmeabführeinheit, insbesondere einen Wärmetauscher, und/oder eine Filtereinrichtung (58) zum Filtern der Flüssigkeit (18) umfasst.

6. Kühlanordnung (10) nach einem der Ansprüche 1 bis 5,
**dadurch gekennzeichnet,**
- **dass** ein Datenspeicher (62) für die Steuereinheit vorgesehen ist, wobei in dem Datenspeicher (62) für ein oder mehrere Nutzungen der Kühlanordnung Daten speicherbar sind, insbesondere zur Temperatur, Nutzungs-/Kühldauerdauer, Nutzungszeitpunkt und/oder Daten mindestens eines Nutzers der Kühlanordnung (10).

7. Kühlanordnung (10) nach Anspruch 6,
**dadurch gekennzeichnet,**
**dass** die Steuereinheit ausgebildet ist, basierend auf im Datenspeicher (62) gespeicherten Daten eine Empfehlung für Eingaben zu einer weiteren oder zukünftigen Nutzungen der Kühlanordnung, insbesondere betreffend Temperatur, Nutzungs-/Kühldauer, Nutzungszeitpunkt, insbesondere betreffend mindestens einen Nutzer, auszugeben, insbesondere über eine Anzeige oder eine vorzugsweise mobile Ausgabeeinrichtung.

8. Kühlanordnung (10) nach einem der Ansprüche 1 bis 7,
**dadurch gekennzeichnet,**
**dass** die Steuereinheit, die Eingabeeinrichtung (42) und/oder die Ausgabeeinrichtung ein Datenverarbeitungsprogramm mit maschinellem Lernen aufweist.

9. Kühlanordnung (10) nach einem der Ansprüche 1 bis 8,
**dadurch gekennzeichnet,**
- **dass** die Kühlanordnung (10) ausgebildet ist, die Flüssigkeit (18) mit Ozon anzureichern.

10. Kühlanordnung (10) nach einem der Ansprüche 1 bis 9,
**dadurch gekennzeichnet,**
- **dass** die Kühlanordnung (10) mindestens eine Zuführung (66) aufweist, die ausgebildet ist, die Flüssigkeit (18) zum Einlass (30) der Kühleinrichtung (12) zu leiten.

11. Kühlanordnung (10) nach Anspruch 10,
**dadurch gekennzeichnet,**
- **dass** die Zuführung (66) mindestens drei Endstücke aufweist, wobei mindestens ein Endstück zum Ableiten der Flüssigkeit (18) aus dem Behältnis (14) ausgebildet ist.

12. Kühlanordnung (10) nach einem der Ansprüche 1 bis 11,
**dadurch gekennzeichnet,**
- **dass** die Kühlanordnung (10) mindestens eine Rückführung (70) aufweist, die ausgebildet ist, Flüssigkeit (18) aus der Kühleinrichtung (12) abzuleiten.

13. Kühlanordnung (10) nach einem der Ansprüche 10 bis 12,
**dadurch gekennzeichnet,**
- **dass** die Rückführung (70) und/oder die Zuführung (66) mindestens ein Fixierelement (74), insbesondere einen Saugnapf, aufweisen.

14. Kühlanordnung (10) nach einem der Ansprüche 1 bis 13,
**dadurch gekennzeichnet,**
- **dass** mindestens ein Koppelelement (78) vorgesehen ist, wobei das Koppelelement (78) zwei Anschlussstücke aufweist, die zum verlustfreien Überleiten der Flüssigkeit (18), insbesondere aus einer Wasserleitungsarmatur (86), ausgebildet sind.

15. Kühlverfahren, insbesondere mit einer Kühlanordnung (10) nach einem der Ansprüche 1 bis 14, wobei
- ein Behältnis (14) zum Aufnehmen eines Körpers, insbesondere einer Wanne (22) oder Tonne (26), mit einer Flüssigkeit (18), insbesondere Wasser, gefüllt wird,
- die Flüssigkeit (18) mit einer Kühleinrichtung (12) mit mindestens einem Einlass (30) für die Flüssigkeit (18) und mindestens einem Auslass (34) für die Flüssigkeit (18) gekühlt wird,
- die Flüssigkeit (18) bei Durchlauf durch die Kühleinrichtung (12) von dem mindestens einen Einlass (30) zu dem mindestens einen Auslass (34) gekühlt wird und von dem Auslass (34) dem Behältnis (14) zugeführt wird, und
- die Kühleinrichtung (12) von einer Steuereinheit zum Einstellen zumindest der Temperatur der Flüssigkeit (18), welche dem Behältnis (14) zugeführt wird, gesteuert wird.

## Geänderte Patentansprüche

### Geänderte Patentansprüche gemäss Regel 137(2) EPÜ.

1. Kühlanordnung (10) mit
- einem Behältnis (14), welches zum Aufnehmen eines Körpers in einer Flüssigkeit (18), insbesondere Wasser, ausgebildet ist, insbesondere eine Wanne (22) oder Tonne (26),
- einer Kühleinrichtung (12), welche zum Kühlen der Flüssigkeit (18) ausgebildet ist und mindestens einen Einlass (30) für die Flüssigkeit (18) und mindestens einen Auslass (34) für die Flüssigkeit (18) aufweist, wobei die Flüssigkeit (18) bei Durchlauf durch die Kühleinrichtung (12) von dem mindestens einen Einlass (30) zu dem mindestens einen Auslass (34) kühlbar ist und von dem Auslass (34) zu dem Behältnis (14) zuführbar ist, und
- einer Steuereinheit zum Steuern der Kühleinrichtung (12) zum Einstellen zumindest der Temperatur der Flüssigkeit (18), welche dem Behältnis (14) zugeführt wird,
**dadurch gekennzeichnet,**
- **dass** ein Datenspeicher (62) für die Steuereinheit vorgesehen ist, wobei in dem Datenspeicher (62) für ein oder mehrere Nutzungen der Kühlanordnung Daten speicherbar sind, zur Temperatur, Nutzungs-/Kühldauer, Nutzungszeitpunkt und Daten mindestens eines Nutzers der Kühlanordnung (10), und
- **dass** die Steuereinheit ausgebildet ist, basierend auf im Datenspeicher (62) gespeicherten Daten eine Empfehlung für Eingaben zu einer weiteren oder zukünftigen Nutzungen der Kühlanordnung betreffend mindestens einen Nutzer auszugeben.

2. Kühlanordnung (10) nach Anspruch 1,
**dadurch gekennzeichnet,**
- **dass** die Steuereinheit mit mindestens einer rechnergestützten, vorzugsweisen mobilen Eingabeeinrichtung (42) zum Eingeben von Daten und/oder Steuerbefehlen verbindbar ist.

3. Kühlanordnung (10) nach Anspruch 2,
**dadurch gekennzeichnet,**
- **dass** die rechnergestützte Eingabeeinrichtung (42) ein Mobiltelefon und/oder einen Tabletcomputer, insbesondere mit einer graphischen Bedienoberfläche, umfasst.

4. Kühlanordnung (10) nach einem der Ansprüche 1 bis 3,
**dadurch gekennzeichnet,**
- **dass** mindestens ein Sensor zum Erfassen von Zuständen, insbesondere von Zustandsdaten des Körpers, vorgesehen ist, wobei der mindestens eine Sensor mit der Steuereinheit und/oder der rechnergestützten Eingabeeinrichtung (42) verbindbar ist.

5. Kühlanordnung (10) nach einem der Ansprüche 1 bis 4,
**dadurch gekennzeichnet,**
- **dass** die Kühleinrichtung (12) eine Wärmeabführeinheit, insbesondere einen Wärmetauscher, und/oder eine Filtereinrichtung (58) zum Filtern der Flüssigkeit (18) umfasst.

6. Kühlanordnung (10) nach einem der Ansprüche 1 bis 5,
**dadurch gekennzeichnet,**
- **dass** die Empfehlung für Eingaben die Temperatur, Nutzungs-/Kühldauer und Nutzungszeitraum betreffen.

7. Kühlanordnung (10) nach einem der Ansprüche 1 bis 6,
**dadurch gekennzeichnet,**
**dass** die Empfehlung für Eingaben über eine Anzeige oder eine mobile Ausgabeeinrichtung ausgebbar ist.

8. Kühlanordnung (10) nach einem der Ansprüche 1 bis 7,
**dadurch gekennzeichnet,**
**dass** die Steuereinheit, die Eingabeeinrichtung (42) und/oder die Ausgabeeinrichtung ein Datenverarbeitungsprogramm mit maschinellem Lernen aufweist.

9. Kühlanordnung (10) nach einem der Ansprüche 1 bis 8,
**dadurch gekennzeichnet,**
- **dass** die Kühlanordnung (10) ausgebildet ist, die Flüssigkeit (18) mit Ozon anzureichern.

10. Kühlanordnung (10) nach einem der Ansprüche 1 bis 9,
**dadurch gekennzeichnet,**
- **dass** die Kühlanordnung (10) mindestens eine Zuführung (66) aufweist, die ausgebildet ist, die Flüssigkeit (18) zum Einlass (30) der Kühleinrichtung (12) zu leiten.

11. Kühlanordnung (10) nach Anspruch 10,
**dadurch gekennzeichnet,**
- **dass** die Zuführung (66) mindestens drei Endstücke aufweist, wobei mindestens ein Endstück zum Ableiten der Flüssigkeit (18) aus dem Behältnis (14) ausgebildet ist.

12. Kühlanordnung (10) nach einem der Ansprüche 1 bis 11,
**dadurch gekennzeichnet,**
- **dass** die Kühlanordnung (10) mindestens eine Rückführung (70) aufweist, die ausgebildet ist, Flüssigkeit (18) aus der Kühleinrichtung (12) abzuleiten.

13. Kühlanordnung (10) nach einem der Ansprüche 10 bis 12,
**dadurch gekennzeichnet,**
- **dass** die Rückführung (70) und/oder die Zuführung (66) mindestens ein Fixierelement (74), insbesondere einen Saugnapf, aufweisen.

14. Kühlanordnung (10) nach einem der Ansprüche 1 bis 13,
**dadurch gekennzeichnet,**
- **dass** mindestens ein Koppelelement (78) vorgesehen ist, wobei das Koppelelement (78) zwei Anschlussstücke aufweist, die zum verlustfreien Überleiten der Flüssigkeit (18), insbesondere aus einer Wasserleitungsarmatur (86), ausgebildet sind.

15. Kühlverfahren, mit einer Kühlanordnung (10) nach einem der Ansprüche 1 bis 14, wobei
- ein Behältnis (14) zum Aufnehmen eines Körpers, insbesondere einer Wanne (22) oder Tonne (26), mit einer Flüssigkeit (18), insbesondere Wasser, gefüllt wird,
- die Flüssigkeit (18) mit einer Kühleinrichtung (12) mit mindestens einem Einlass (30) für die Flüssigkeit (18) und mindestens einem Auslass (34) für die Flüssigkeit (18) gekühlt wird,
- die Flüssigkeit (18) bei Durchlauf durch die Kühleinrichtung (12) von dem mindestens einen Einlass (30) zu dem mindestens einen Auslass (34) gekühlt wird und von dem Auslass (34) dem Behältnis (14) zugeführt wird, und
- die Kühleinrichtung (12) von einer Steuereinheit zum Einstellen zumindest der Temperatur der Flüssigkeit (18), welche dem Behältnis (14) zugeführt wird, gesteuert wird.
